(19) Europäisches Patentamt
European Patent Office
Office européen des brevets

(11) **EP 3 357 411 A1**

(12) **EUROPEAN PATENT APPLICATION**

(43) Date of publication:
**08.08.2018 Bulletin 2018/32**

(51) Int Cl.:
**A61B 5/00** *(2006.01)*    **A61B 5/021** *(2006.01)*
**A61B 5/024** *(2006.01)*    **A61B 5/1455** *(2006.01)*

(21) Application number: **17154941.3**

(22) Date of filing: **07.02.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Koninklijke Philips N.V.**
**5656 AE Eindhoven (NL)**

(72) Inventor: **PRESURA, Cristian Nicolae**
**5656 AE Eindhoven (NL)**

(74) Representative: **de Haan, Poul Erik**
**Philips International B.V.**
**Philips Intellectual Property & Standards**
**High Tech Campus 5**
**5656 AE Eindhoven (NL)**

(54) **OPTICAL SENSING APPARATUS AND CORRESPONDING OPTICAL SENSING METHOD**

(57)    An optical sensing apparatus (10), a corresponding optical sensing method and a corresponding computer program configured to sense a property of a living being are provided. The optical sensing apparatus (10) comprises a contact surface (20) configured to be brought into contact with the skin (5) of the living being, a first optical sensor unit (30; 630) for providing a first optical signal and a second optical sensor unit (40; 640) for providing a second optical signal, wherein the first optical sensor unit (30; 630) and the second optical sensor unit (40; 640) are arranged on the contact surface (20) at a distance from each other, wherein the first optical sensor unit (30; 630) has a different protrusion length (50) from the contact surface (20) than the second optical sensor unit (40, 640). The optical sensing apparatus (10) allows a more reliable determination of a property of a living being.

FIG. 7

EP 3 357 411 A1

**Description**

FIELD OF THE INVENTION

The present invention relates to an optical sensing apparatus, a corresponding optical sensing method and a corresponding computer program. The invention relates in particular to an optical sensing apparatus configured to sense a property of a living being in contact with a skin of the living being. In a preferred application, the optical sensing apparatus is an optical heart monitor, wherein the optical sensing apparatus according to the invention is not limited to this application.

BACKGROUND OF THE INVENTION

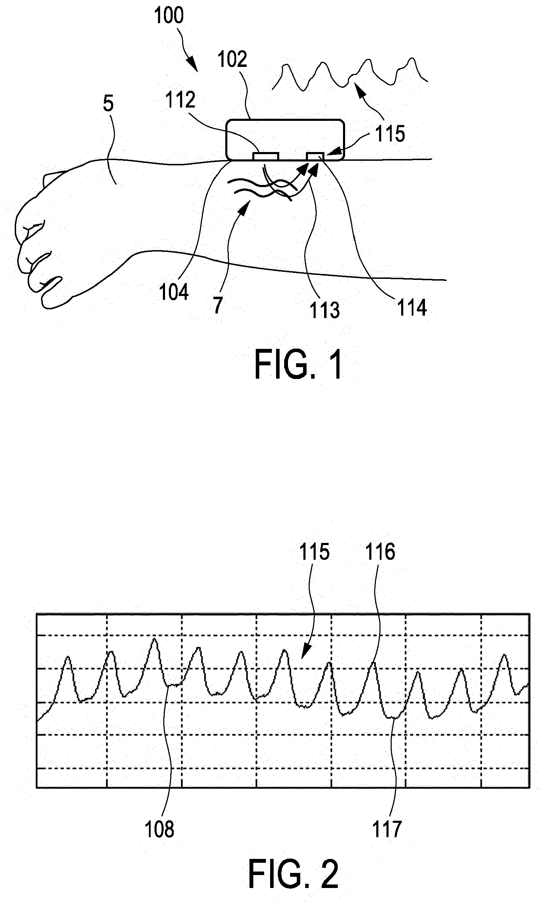

The principles of an optical sensing apparatus are illustrated for the example of an optical heart monitor 100 in Fig. 1, in which the sensed property exemplarily corresponds to a heart rate. Optical heart monitor 100 comprises a casing 102 having a contact surface 104 which is arranged in contact with a skin 5 of a living being, for instance an arm or wrist of a human being. The optical heart monitor 100 comprises an optical sensor unit 110 disposed at contact surface 104 and including a light emitting component 112 and a light detection component 114. Light detection component 112, for instance a light emission diode (LED), shines light 113 inside skin 5, the light is then scattered in the skin 5, where it is absorbed more or less by blood circulating through blood vessels 7. Light 113 exits the skin 5 and enters light detection component 114, for instance a photodiode. Since the blood volume in the skin 5 changes when the heart pulsates, the amount of light absorbed changes over time and a signal 115 on the light detection component 114 changes accordingly. Signal 115 of optical heart monitor 100 is therefore indicative of a pulse in the skin 5 and thus the heart rate of the living being.

Signal 115 is exemplarily illustrated in further detail in Fig. 2. A difference between a local maximum 116 and a local minimum 117 is called an amplitude of the signal. For instance by counting the number of maxima 116 per second, the number of heartbeats per minute can be obtained in the example of optical heart monitors. Fig. 2 illustrates signal 115 while the subject is not in motion, i.e. substantially at rest. Nevertheless, a difference in offset between two different minima 117 and 118 is already noticeable.

From US 4,880,304 and US 6,622,034 it is known that the amplitude of the pulse of the optical signal depends on a protrusion of the sensor in the skin, especially for small sensors. A larger protrusion allows for an increased amplitude and therefore for a facilitated signal processing.

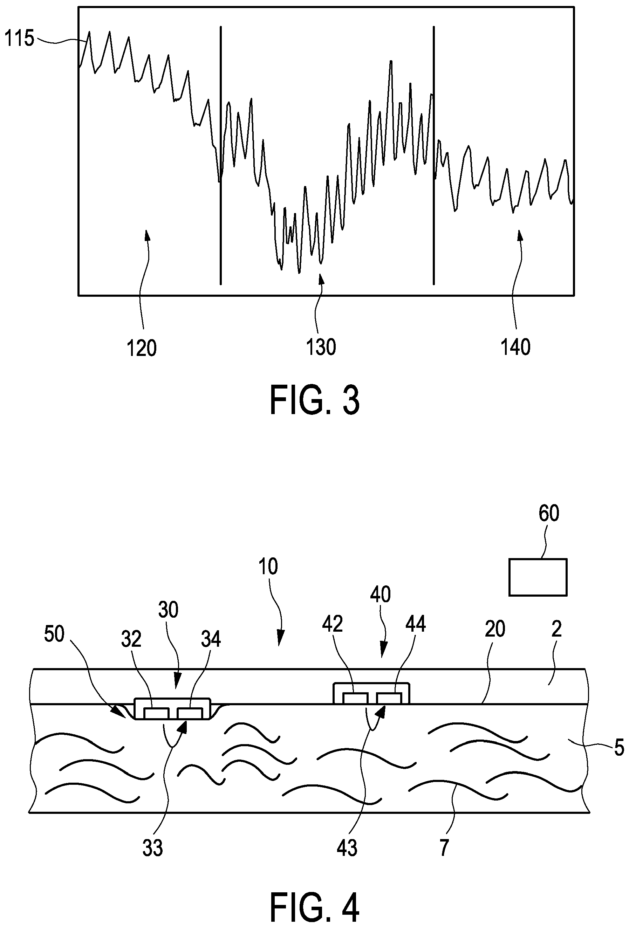

However, in particular during motion the signal is corrupted with motion artifacts, which influence both amplitude and offset of signal 115, as exemplarily depicted in Fig. 3. Therein, a still period 120 is followed by a motion period 130 and another still period 140. During the still period 120, the pulse is clearly visible and heart rate extraction is easily possible. However, the determination of a property, such as a heart rate, from signal 115 during the motion period 130 can be unreliable due to the motion artifacts.

SUMMARY OF THE INVENTION

It has therefore been an object of the present invention to provide an optical sensing apparatus which allows a more reliable determination of a property of a living being.

In one aspect an optical sensing apparatus configured to sense a property of a living being in contact with a skin of the living being is provided. The optical sensing apparatus comprises a contact surface configured to be brought into contact with the skin of the living being, a first optical sensor unit for providing a first optical signal and a second optical sensor unit for providing a second optical signal. The first optical sensor unit and the second optical sensor unit are arranged on the contact surface at a distance from each other, wherein the first optical sensor unit has a different protrusion length from the contact surface than the second optical sensor unit.

Since the first optical sensor unit and the second optical sensor unit have a different protrusion length from the contact surface, the first optical sensor unit and the second optical sensor unit protrude differently into the skin of the living being when the contact surface is brought into contact with the skin of the living being. Since different protrusion depths result in different amplitudes of pulses of the optical signal, the property of the living being can be extracted from the first optical signal and the second optical signal more reliably since both signals comprise the same pulse signal with respectively different amplitude.

The property of the living being is preferably at least one of a heart rate, a blood oxygenation (SpO$_2$) and a blood pressure. However, also other properties of interest of the living being are of course feasible.

Preferably, the contact surface is substantially planar. Thereby an equal pressure distribution on the skin by the contact surface and relative thereto a defined pressure and protrusion into the skin resulting from the protrusion from the contact surface can be ensured. At least one of the first optical sensor unit and the second optical sensor unit protrude from the contact surface to have a different protrusion into the skin than the rest of the contact surface. In case both the first optical sensor unit and the second optical sensor unit protrude from the contact surface, their respective protrusion lengths are preferably different. In other embodiments, the contact surface can also be formed to replicate a surface shape of a part of a body of the living being, such as a wrist.

In one embodiment each of the first and second optical sensor units comprises a light emitting component and a light detection component.

The light emitting component is arranged for emitting light into the skin, which gets scattered in the skin, is partly absorbed and then partly exits the skin and enters the light detection component. Preferably, the light emitting component comprises at least one light emitting diode (LED) and/or the light detection component comprises at least one photodiode (PD). In other embodiments, also other light emitting components and/or light detection components are of course contemplated.

In one embodiment the light emitting component and the light detection component of the first and second optical sensor units are arranged at the same distance from each other, respectively.

A distance between light detection component and light emitting component is indicative of a tissue sampling depth, i.e. a depth up to which a photon emitted by the light emitting component has most probably penetrated into the skin upon its path of scattering. The first optical sensor unit and the second optical sensor unit thus sample optical signals with corresponding tissue depths from the surface of the respective sensor unit in this embodiment. Accordingly, the sampled tissue depths of the first optical sensor unit and the second optical sensor unit are comparable, which makes their respective signals suitable for determining the same property of the living being.

In one embodiment at least one of the light emitting component and the light detection component, preferably both the light emitting component and the light detection component, of the first optical sensor unit has a different protrusion from the second optical sensor unit.

Preferably, the light emitting component and the light detection component have a same protrusion into the skin such that photons migrate the same distance into the skin as out of the skin. In case the light emitting component has a different protrusion from the light detection component, it is preferred that the same distance in protrusion also applies to the other sensor unit of the first optical sensor unit and the second optical sensor unit. Thereby a tissue sampling depth being equal among the first optical sensor unit and the second optical sensor unit can be ensured.

In one embodiment the optical sensing apparatus further comprises a casing, wherein the contact surface is formed on a surface of the casing.

In one embodiment the optical sensing apparatus is provided in the form of a wrist-worn device. Preferably, the wrist-worn device comprises a watch, which is arranged for being attached to a wrist of the living being by means of a strap or the like. Thereby, closed contact of the contact surface to the skin of the living being can be ensured. However, also other forms and shapes of devices for the optical sensing apparatus are of course contemplated to a person skilled in the art, such as including chest belts, in-ear plugs and the like.

In one embodiment the optical sensing apparatus further comprises a property determination unit for determining the property of the living being based on a difference between the first optical signal and the second optical signal.

The property determination unit preferably comprises a processing unit such as a processor. The property determination unit is arranged to receive the first optical signal and the second optical signal from the first optical sensor unit and the second optical sensor unit, respectively. In case of an embodiment comprising a casing and/or the optical sensing apparatus being provided in the form of wrist-worn device, the property determination unit can be provided within the casing and/or the wrist-worn device or at a different location. In one embodiment the property determination unit can be implemented on a remote unit, such as a smart phone, a server and the like, and be arranged for receiving the first optical signal and the second optical signal or a processed form thereof from the first optical sensor unit and the second optical sensor unit through wired or wireless transmission.

The first optical signal and the second optical signal contain the pulse with a different amplitude, respectively, as discussed above. Since motion artifacts can be assumed to be similar among the first optical signal and the second optical signal, by determining the property based on a difference between both signals the motion artifacts comprised in both signals become illuminated. Accordingly, the resulting difference is advantageously devoid of motion artifacts and only contains contributions which are indicative of the property to be determined.

In one embodiment the property determination unit is configured to determine the property further based on a factor depending on the protrusion length.

It can be assumed that both the first optical signal and the second optical signal comprise a pulse component, respectively scaled with a factor depending on the protrusion, and a motion artifact or noise component. A difference between the first optical signal and the second optical signal thus results in the pulse signal multiplied by a difference between the two factors dependent on the protrusion. By estimating and eliminating, for instance, the factor depending on the protrusion, the property can be determined more accurately.

In one embodiment the first optical sensor unit comprises a lens.

In this embodiment, the lens preferably forms the protrusion from the contact surface. Preferably, the lens is attached to, such as glued on, the contact surface. In other examples, different elements can form the protrusion of the first and/or second optical sensor unit. In other embodiments, both the first and second optical sensor unit comprise a lens, which is preferably attached on the contact surface, wherein the respectively attached lenses have different sizes, such as to ensure the different protrusion between the first optical sensor unit and the second optical sensor unit.

In one embodiment at least one of the first optical sensor unit and the second optical sensor unit is formed as a spectral optical sensor unit.

Spectral optical sensors allow the determination of an optical signal for at least two different wavelengths of light emitted into the skin. Spectral optical sensor units can either be full spectra sensor units, i.e. which are capable of determining a full spectrum or a broader range between two wavelengths, or are discrete spectra sensor units, i.e. configured to obtain the optical signal for a particular number of different discrete wavelengths, such as two or three, etc. Preferably, the at least one of the first optical sensor unit and the second optical sensor unit comprises a plurality of light emitting components, such as LEDs of different wavelengths. However, other examples of spectral optical sensor units are of course contemplated.

In one embodiment both the first optical sensor unit and the second optical sensor unit are formed as spectral optical sensor units for providing a first and second optical spectrum, respectively. The optical sensing apparatus further comprises a pulse oxygenation determination unit for determining an oxygenation of venous blood. The pulse oxygenation determination unit is configured to determine the blood oxygenation based on a difference of the first and second optical spectrum.

A blood pressure of venous blood is significantly lower than blood pressure of, for instance, arterial blood. Since one of the first optical sensor unit and the second optical sensor unit protrudes further from the contact surface than the other, pressure applied on the skin is higher for the more protruding optical sensor unit. Thereby, venous blood is pushed away from the optical sensor unit having the longer protrusion length. Accordingly, only the optical sensor unit with less protrusion is expected to provide an optical signal indicative of venous blood. Thereby, by subtracting the two spectra of the first and second optical sensor unit, the spectrum of the venous blood is directly obtained, of which the blood oxygenation can be determined. For instance, the blood oxygenation can be determined by comparing an absorption of two different components of the spectra, as known in the art.

In one embodiment the first optical sensor unit and the second optical sensor unit are configured to be provided along an artery of the living being. The optical sensing apparatus further comprises a blood pressure determination unit for determining a blood pressure of the living being based on a difference of pulse shapes of the first optical signal and the second optical signal.

The sensor unit out of the first optical sensor unit and second optical sensor unit having the longer protrusion depth applies a higher pressure onto the skin and thus onto the artery of the living being. Thereby, a local cuff is formed which blocks at least part of the underlying blood vessels. Since the artery on which the optical sensing apparatus is provided is compressed, the amplitude and shape of the pulse of the optical signal will be different between the first optical sensor unit and the second optical sensor unit. The difference in pulse shapes of the first optical signal and the second optical signal is indicative of blood pressure and stiffness in a favorable way.

In a further aspect an optical sensing method for sensing a property of a living being in contact with a skin of the living being is provided. The method comprises contacting a contact surface of an optical sensing apparatus with the skin of the living being and providing a first optical signal using a first optical sensor unit and a second optical signal using a second optical sensor unit, wherein the first optical sensor unit and the second optical sensor unit are arranged on the contact surface at a distance from each other, wherein the first optical sensor unit has a different protrusion length from the contact surface than the second optical sensor unit.

In a further aspect a computer program is provided. The computer program comprises program code means for causing an optical sensing apparatus as defined in claim 1 to carry out at least parts of the optical sensing method as defined in claim 14, when the computer program is run on the optical sensing apparatus.

It shall be understood that the optical sensing apparatus of claim 1, the optical sensing method of claim 14, and the computer program of claim 15 have similar and/or identical preferred embodiments, in particular, as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

BRIEF DESCRIPTION OF THE DRAWINGS

In the following drawings:

Fig. 1 schematically and exemplarily illustrates an optical heart monitor according to the state of the art,

Fig. 2 schematically and exemplarily illustrates a signal of the optical heart monitor,
Fig. 3 schematically and exemplarily illustrates the signal of Fig. 2 during periods of rest and motion,
Fig. 4 schematically and exemplarily illustrates a first example of an optical sensing apparatus according to the invention,
Fig. 5 schematically and exemplarily illustrates signals of the optical sensing apparatus of Fig. 4,
Fig. 6 schematically and exemplarily illustrates a second example of an optical sensing apparatus according to the invention,
Fig. 7 schematically and exemplarily illustrates a third example of an optical sensing apparatus according to the invention, and
Fig. 8 schematically and exemplarily illustrates a flowchart of an embodiment of an optical sensing method.

DETAILED DESCRIPTION OF EMBODIMENTS

Fig. 4 shows schematically and exemplarily an optical sensing apparatus 10 according to an embodiment of the present invention. Optical sensing apparatus 10 comprises a casing 2 having a contact surface 20 in contact with a skin 5 of a living being. Further, a first optical sensor unit 30 and a second optical sensor unit 40 are provided on the contact surface 20 side of optical sensing apparatus 10 in contact with skin 5.

In this example, first optical sensor unit 30 and second optical sensor unit 40 comprise a light emitting component 32 and 42 each. Light 33, 43 emitted by light emitting component 32, 42 is scattered within skin 5 and exits skin 5 to enter a light detection component 34, 44 of the first optical sensor unit 30 and the second optical sensor unit 40, respectively. While being scattered within the skin, light 33, 43 is absorbed more or less depending on how much blood 7 is present in the skin 5 volume underneath the respective sensor unit. The amount of light entering the respective light detection component 34, 44 is an indication of the blood volume in the skin. Due to the pulsating of the heart of the living being, the blood volume in the skin changes and the signal of the respective light detection component 34, 44 changes accordingly.

First optical sensor unit 30 in this example protrudes further from contact surface 20 than second optical sensor unit 40 by a protrusion length 50. In this example, the first optical sensor unit 30 therefore protrudes further into the skin 5 and will provide a signal indicative of the pulsating heart with a higher amplitude. The protrusion length 50 is in this example achieved by directly providing first optical sensor unit 30 protruding from contact surface 20. In other examples, additionally or alternatively to providing the first optical sensor unit 30 protruding from contact surface 20, a lens can be attached to contact surface 20 on top of at least one of the sensor units to attain the desired protrusion.

In the following, $s_1(t)$ will be referred to as a signal obtained by light detection component 34 and $s_2(t)$ will be referred to as a signal of light detection component 44. A pulse $p(t)$, which is indicative of a volume of blood underneath the sensor and can be used for determining the property of the living being, such as the heart rate, a blood oxygenation concentration or a blood pressure, is assumed to be comprised in both sensor signals $s_1(t)$ and $s_2(t)$ with a different amplitude, respectively. Further, motion artifacts, indicated as $m(t)$, are assumed to be the same for both optical sensor units 30, 40 both in shape and amplitude.

For example, the signals $s_1$ and $s_2$ determined by light detection component 34, 44 can then be written as follows:

$$s_1(t) = a_1 * p(t) + m(t);$$

$$s_2(t) = a_2 * p(t) + m(t).$$

$a_1$ and $a_2$ are factors dependent on the protrusion. $a_1$ and $a_2$ or a difference between $a_1$ and $a_2$ can be estimated and the pulse $p(t)$ be determined as follows:

$$p(t) = \frac{s_1(t) - s_2(t)}{a_1 - a_2}.$$

In this example optical sensing apparatus 10 comprises a property determination unit 60 for determining the property of the living being from the signals $s_1$ and $s_2$, for instance according to the formula provided above. Property determination unit 60 can comprise a processing unit, such as a CPU, and can be provided within casing 2 or distant therefrom, such as at a remote device including a smartphone (not shown), a server (not shown) and the like. In the latter example, it is preferred that the signals $s_1$ and $s_2$ be transferred to property determination unit 60 by any suitable wired or wireless connection as known in the art.

In this example, light emitting component 32 and/or 42 comprises a light emitting diode (LED), which emits, for instance, green light. Light detection component 34 and/or 44 comprises in this example a photodiode (PD) configured to receive scattered light. However, also other examples of light emitting component 32, 42 and/or light detection component 42, 44 as known to the skilled person can be used instead.

In this example, the distance between light emitting component and light detection component is the same among the first optical sensor unit 30 and the second optical sensor unit 40. In other examples, the distance between light emitting component and corresponding light detection component can also be different.

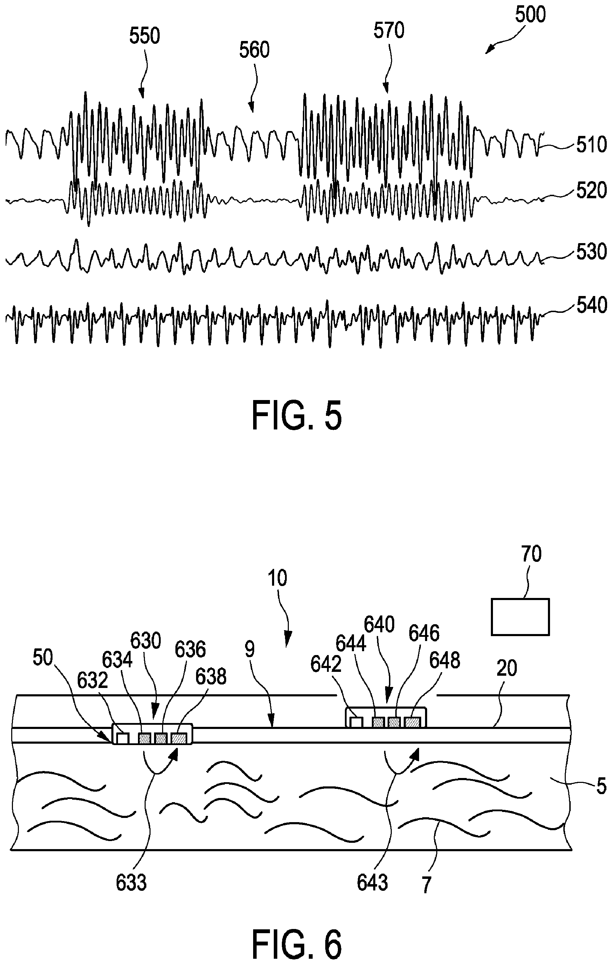

Sensor signals measured by optical sensing apparatus 10 are exemplarily illustrated over a particular time in Fig. 5. Fig. 5 illustrates a plot 500 which displays signal sequences 510, 520, 530 and 540 over time. In this example, sequence 510 represents signal $s_2$, i.e. the signal measured by second optical sensor unit 40. Sequence 520 represents signal $s_1$, i.e. the signal of first optical sensor unit 30 protruding into the skin. During the measuring period visible in Fig. 5, two periods with motion, indicated with 550 and 570, surround a period without motion, indicated with 560. It can be seen that both sequences 510 and 520 taken individually have difficulties to be indicative of the pulse particularly in the periods of motion 550 and 570.

Sequence 530 represents a linear combination of signals 510 and 520 and shows an improved sequence of consecutive pulses. It is obvious that a property of the living being, e.g. the heart rate, can be determined more reliably from sequence 530 than from any of the underlying signals 510 and 520 alone. Sequence 540 illustrates an electrocardiograph (ECG) signal as a reference.

Fig. 6 shows a further embodiment of an optical sensing apparatus 10 according to the invention. In this example, compared to the embodiment shown in Fig. 4, a first optical sensor unit 630 and a second optical sensor unit 640 are provided with spectroscopic capabilities, such as for determining arterial oxygen saturation ($SpO_2$) as a property of the living being. Each of the first and second optical sensor unit 630, 640 therefore comprises multiple light emitting components, in this example indicated with 632, 634, 636, 642, 644 and 646. The number of individual light emitting components is of course exemplary, and other numbers of light emitting components can be used in other examples. Preferably, each of the first and second optical sensor unit comprises LEDs of three different colors, such as green, blue and red. In other examples it is preferred that each of the first second optical sensor unit comprise a red LED and an infrared LED. Both optical sensor units comprise a photodiode 638, 648 as an example for a light detection component. First optical sensor unit 630 protrudes about a protrusion length 50 further from contact surface 20 and into skin 5 then second optical sensor unit 640. Thereby, venous blood 9, which has a lower pressure than arterial blood, is pushed away by first optical sensor unit 630. In this way, by subtracting spectra of first optical sensor unit 630 and second optical sensor unit 640, the spectrum of the venous blood, which is indicative of the amount of oxygenated hemoglobin in blood, can directly be measured. This is illustrated in Fig. 6 by exemplary light migration routes 633 and 643, wherein light 633 does not pass through venous blood 9, which accumulates close to a surface of skin 5, while light 643 to the contrary passes through venous blood 9. To this end, in the example of Fig. 6 optical sensing apparatus 10 comprises a pulse oxygenation determination unit 70 which is arranged for determining the amount of oxygenated hemoglobin in blood from the determined spectra as known in the art. Pulse oxygenation determination unit 70 can be combined with property determination unit 60 and/or provided as a separate unit, wherein pulse oxygenation determination unit 70 can likewise be provided within the casing or distant therefrom.

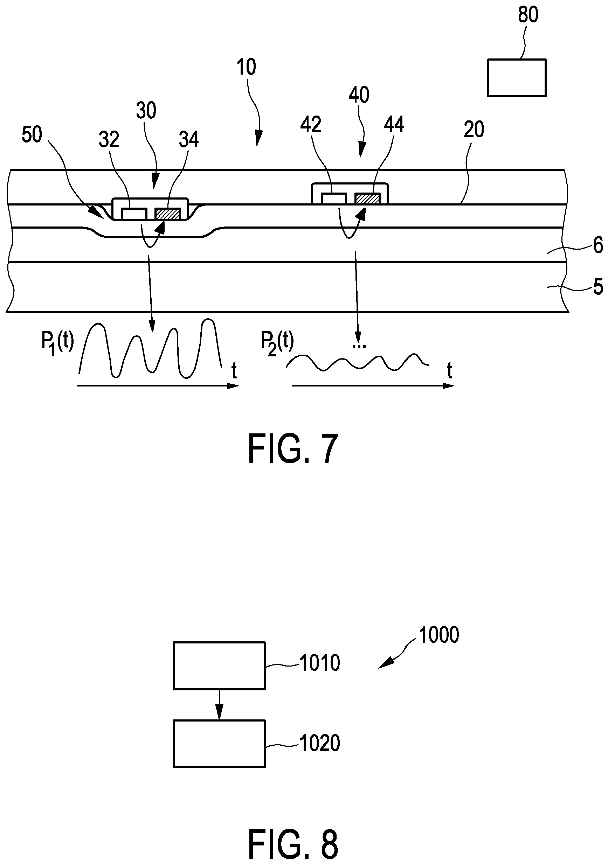

Fig. 7 schematically and exemplarily illustrates a further example of optical sensing apparatus 10 in the signal light emitting component configuration as shown in Fig. 4. However, it is obvious that this example can also be combined with the spectral example illustrated in Fig. 6. In this example, optical sensing apparatus 10 is provided on top of a large artery 6. In a region, in which the first optical sensor unit 30 which protrudes about protrusion length 50 from contact surface 20 is disposed, artery 6 shows a region 8 in which a diameter is reduced, corresponding to a compression of artery 6. Preferably, an amount and the way in which the blood will flow can be given by the protrusion length 50 and a local geometry of the tissue. $p_1(t)$ indicates light received by first light detection component 34 and $p_2(t)$ indicates a signal received by second light detection component 44. Both are illustrated schematically over a certain period of time $t$. It can be determined that both a shape and an amplitude is different between $p_1(t)$ and $p_2(t)$, wherein the difference allows obtaining information about the blood pressure and stiffness at the region of optical sensing apparatus 10, which can be represented as a function depending on blood pressure and stiffness:

$$p_1(t) - p_2(t) = f\left(blood\ pressure, stiffness\right).$$

The functional dependence then allows determining blood pressure and stiffness based on the received signals $p_1(t)$ and $p_2(t)$ by a blood pressure determination unit 80. Blood pressure determination unit 80 can be combined with at least one of property determination unit 60 and blood oxygenation determination unit 70 and/or be provided as a separate unit, wherein blood pressure determination unit 80 can likewise be provided within the casing or distant therefrom.

Fig. 8 shows schematically and exemplarily an optical sensing method 1000 for sensing a property of a living being in contact with a skin of the living being. Optical sensing method 1000 comprises the step 1010 of providing a contact surface 20 of an optical sensing apparatus in contact with the skin 5 of the living being, wherein optical sensing apparatus 10 of any of the examples of the disclosure can be used for instance.

Next, optical sensing method 1000 comprises a step 1020 of providing a first optical signal using a first optical sensor unit 30, 630 and a second optical signal using a second optical sensor unit 40, 640, wherein the first optical sensor unit 30, 630 and the second optical sensor unit 40, 640 are arranged on the contact surface 20 at a distance from each other, wherein the first optical sensor unit 30, 630 has a different protrusion length from the contact surface 20 than the second optical sensor unit 40, 640.

At least some of the steps of the method can preferably be implemented as a computer program to be executed on at least one of property determination unit 60, pulse oxygenation determination unit 70 and blood pressure determination unit 80.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.

## Claims

1. An optical sensing apparatus configured to sense a property of a living being in contact with a skin (5) of the living being, comprising
   a contact surface (20) configured to be brought into contact with the skin (5) of the living being,
   a first optical sensor unit (30; 630) for providing a first optical signal and a second optical sensor unit (40; 640) for providing a second optical signal, wherein the first optical sensor unit (30; 630) and the second optical sensor unit (40; 640) are arranged on the contact surface (20) at a distance from each other,
   wherein the first optical sensor unit (30; 630) has a different protrusion length (50) from the contact surface (20) than the second optical sensor unit (40, 640).

2. The optical sensing apparatus according to claim 1, wherein the property is at least one of a heart rate, a blood oxygenation and a blood pressure.

3. The optical sensing apparatus according to claim 1, wherein each of the first (30; 630) and second (40; 640) optical sensor units comprises a light emitting component (32, 42; 632, 634, 636, 642, 644, 646) and a light detection component (34, 44; 638, 648).

4. The optical sensing apparatus according to claim 3, wherein the light emitting component (32, 42; 632, 634, 636, 642, 644, 646) and the light detection component (34, 44; 638, 648) of the first (30; 630) and second (40; 640) optical sensor units are arranged at the same distance from each other, respectively.

5. The optical sensing apparatus according to claim 3, wherein at least one of the light emitting component (32, 42; 632, 634, 636, 642, 644, 646) and the light detection component (34, 44; 638, 648), preferably both the light emitting component (32, 42; 632, 634, 636, 642, 644, 646) and the light detection component (34, 44; 638, 648), of the first optical sensor unit (30; 630) has a different protrusion from the second optical sensor unit (40; 640).

6. The optical sensing apparatus according to claim 1, further comprising a casing (2), wherein the contact surface (20) is formed on a surface of the casing.

7. The optical sensing apparatus according to claim 1, wherein the optical sensing apparatus (10) is provided in the form of a wrist-worn device.

8. The optical sensing apparatus according to claim 1, further comprising a property determination unit (60) for determining the property of the living being based on a difference between the first optical signal and the second optical signal.

9. The optical sensing apparatus according to claim 8, wherein the property determination unit (60) is configured to determine the property further based on a factor depending on the protrusion length (50).

10. The optical sensing apparatus according to claim 1, wherein the first optical sensor unit (30; 630) comprises a lens.

11. The optical sensing apparatus according to claim 1, wherein at least one of the first optical sensor unit (630) and the second optical sensor unit (640) is formed as a spectral optical sensor unit.

12. The optical sensing apparatus according to claim 11, wherein both the first optical sensor unit (630) and the second optical sensor unit (640) are formed as spectral optical sensor units for providing a first and second optical spectrum, respectively, wherein the optical sensing apparatus (10) further comprises a pulse oxygenation determination unit (70) for determining an oxygenation of venous blood, wherein the pulse oxygenation determination unit (70) is configured to determine the blood oxygenation based on a difference of the first and second optical spectrum.

13. The optical sensing apparatus according to claim 1, wherein the first optical sensor unit (30; 630) and the second optical sensor unit (40; 640) are configured to be provided along an artery (6) of the living being, the optical sensing apparatus (10) further comprising a blood pressure determination unit (80) for determining a blood pressure of the living being based on a difference of pulse shapes of the first optical signal and the second optical signal.

14. An optical sensing method for sensing a property of a living being in contact with a skin of the living being, comprising providing (1010) a contact surface (20) of an optical sensing apparatus in contact with the skin (5) of the living being, providing (1020) a first optical signal using a first optical sensor unit (30; 630) and a second optical signal using a second optical sensor unit (40; 640), wherein the first optical sensor unit (30; 630) and the second optical sensor unit (40; 640) are arranged on the contact surface (20) at a distance from each other, wherein the first optical sensor unit (30; 630) has a different protrusion length from the contact surface (20) than the second optical sensor unit (40; 640).

15. A computer program comprising program code means for causing an optical sensing apparatus as defined in claim 1 to carry out at least parts of the optical sensing method (1000) as defined in claim 14, when the computer program is run on the optical sensing apparatus (10).

FIG. 1

FIG. 2

FIG. 3

FIG. 4

FIG. 5

FIG. 6

$P_1(t)$

$P_2(t)$

**FIG. 7**

**FIG. 8**

Europäisches
Patentamt

European
Patent Office

Office européen
des brevets

# EUROPEAN SEARCH REPORT

Application Number

EP 17 15 4941

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2016/270676 A1 (YAMASHITA HIDETO [JP]) 22 September 2016 (2016-09-22) <br> * paragraph [0070] - paragraph [0075]; figure 3 * <br> * paragraph [0078] - paragraph [0092]; figure 4 * <br> * paragraph [0098] - paragraph [0100]; figures 6,7 * <br> * paragraph [0128] - paragraph [0130]; figure 10 * <br> * paragraph [0133] - paragraph [0137]; figure 11 * <br> * paragraph [0138] - paragraph [0142]; figure 12 * <br> * paragraph [0143] - paragraph [0147]; figure 13 * <br> * paragraph [0148] - paragraph [0152]; figure 14 * <br> * paragraph [0240] * | 1-15 | INV. <br> A61B5/00 <br> A61B5/021 <br> A61B5/024 <br> A61B5/1455 |
| X <br><br> A | WO 2015/146138 A1 (SEIKO EPSON CORP [JP]) 1 October 2015 (2015-10-01) <br> * paragraph [0048] - paragraph [0050]; figure 4 * <br> * paragraph [0076]; figure 1A * <br> * paragraph [0091] - paragraph [0092]; figure 11 * <br> * paragraph [0117] - paragraph [0120]; figure 17 * <br> * paragraph [0127] - paragraph [0131] * | 1-3,6-15 <br><br> 4,5 | **TECHNICAL FIELDS SEARCHED (IPC)** <br> A61B |

The present search report has been drawn up for all claims

| Place of search | Date of completion of the search | Examiner |
|---|---|---|
| The Hague | 1 August 2017 | Weiss-Schaber, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 17 15 4941

This annex lists the patent family members relating to the patent documents cited in the above-mentioned European search report.
The members are as contained in the European Patent Office EDP file on
The European Patent Office is in no way liable for these particulars which are merely given for the purpose of information.

01-08-2017

| Patent document cited in search report | Publication date | Patent family member(s) | Publication date |
|---|---|---|---|
| US 2016270676 A1 | 22-09-2016 | JP 2016174685 A<br>US 2016270676 A1 | 06-10-2016<br>22-09-2016 |
| WO 2015146138 A1 | 01-10-2015 | CN 104939812 A<br>EP 3122245 A1<br>JP 2015188498 A<br>US 2017112398 A1<br>WO 2015146138 A1 | 30-09-2015<br>01-02-2017<br>02-11-2015<br>27-04-2017<br>01-10-2015 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4880304 A
- US 6622034 B